Europäisches Patentamt

European Patent Office

Office européen des brevets

(11) Publication number: **0 418 974 A1**

# EUROPEAN PATENT APPLICATION

(21) Application number: **90202473.6**

(51) Int. Cl.⁵: **C07C 2/84**

(22) Date of filing: **18.09.90**

(30) Priority: **19.09.89 US 409369**

(43) Date of publication of application:
**27.03.91 Bulletin 91/13**

(84) Designated Contracting States:
**AT BE CH DE DK ES FR GB GR IT LI LU NL SE**

(71) Applicant: **UNION CARBIDE CHEMICALS AND PLASTICS COMPANY, INC.**
**39 Old Ridgebury Road**
**Danbury Connecticut 06817-0001(US)**

(72) Inventor: **Warren, Barbara Knight**
**1620 Woodvale Drive**
**Charleston, West Virginia 25314(US)**

(74) Representative: **Smulders, Theodorus A.H.J., Ir. et al**
**Vereenigde Octrooibureaux Nieuwe Parklaan 107**
**NL-2587 BP 's-Gravenhage(NL)**

(54) **Silver-containing catalysts for oxidative coupling.**

(57) Oxidative coupling of lower alkane to higher hydrocarbon is conducted using catalyst comprising at least one of alkali metal and alkaline earth metal and an activity-enhancing amount of silver in the presence of halogen component.

EP 0 418 974 A1

## SILVER-CONTAINING CATALYSTS FOR OXIDATIVE COUPLING

This invention was made under United States of America Government support under Contract No. DE-AC22-87PC79817 awarded by the Department of Energy. The Government has certain rights in this invention.

This invention relates to catalysts and processes for using the catalysts for the oxidative coupling of lower molecular weight alkane to higher molecular weight hydrocarbons.

Background of the Invention

Processes for the conversion of lower molecular weight alkanes such as methane to higher molecular weight hydrocarbons which have greater value are sought. One of the proposals for the conversion of lower molecular weight alkanes is by oxidative coupling. For instance, G. E. Keller and M. M. Bhasin disclose in Journal of Catalysis , Volume 73, pages 9 to 19 (1982) that methane can be converted to, e.g., ethylene. The publication by Keller, et al., has preceded the advent of substantial patent and open literature disclosures by numerous researchers pertaining to processes for the oxidative coupling of lower alkanes and catalysts for such processes.

In order for an oxidative coupling process to be commercially attractive, the process should be capable of providing a good rate of conversion of the lower alkanes with high selectivity to the sought higher molecular weight hydrocarbons. Since conversion and selectivity can be enhanced by catalysts, catalytic processes have been the thrust of work done by researchers in oxidation coupling.

Even using catalysts, high temperatures have been required to achieve desirable conversions of hydrocarbons in oxidative coupling processes. The high temperatures used in oxidative coupling require special materials of construction and may adversely affect stability of the catalyst. Accordingly, catalysts are sought that exhibit enhanced activities without deleterious effects on the selectivities to higher hydrocarbons.

Two general types of oxidative coupling processes are the sequential, or pulsed, processes and the cofeed processes. The sequential processes are characterized by alternately cycling an oxygen-containing gas and an alkane-containing gas for contact with a catalyst. These processes typically provide high selectivities to higher hydrocarbon but suffer from operational complexities in cycling the catalyst environment and in the tendency of the processes to produce less desirable, higher molecular weight products and to have carbonaceous deposits form on the catalyst, thereby necessitating regeneration. Thus, from an operational standpoint, cofeed processes, i.e., processes in which oxygen-containing material and alkane are simultaneously fed to the reaction zone containing the catalyst, are more desirable.

In order for cofeed processes to be commercially attractive, especially for the production of large volume commodity chemicals such as ethylene and ethane ($C_2$'s), not only should the conversion of alkane and the selectivity to higher hydrocarbons as opposed to combustion products such as carbon dioxide and carbon monoxide be high, but also, the catalyst exhibit a relatively long lifetime with high performance.

Numerous catalysts have been proposed by researchers for oxidative coupling processes. These catalysts have included catalysts containing alkali and/or alkaline earth metals. The alkali and alkaline earth metals have been suggested as being in the oxide, carbonate and halide forms. Other components such as rhenium, tungsten, copper, bismuth, lead, tin, iron, nickel, zinc, indium, vanadium, palladium, platinum, iridium, uranium, osmium, rhodium, zirconium, titanium, lanthanum, aluminum, chromium, cobalt, beryllium, germanium, antimony, gallium, manganese, yttrium, cerium, praseodymium (and other rare earth oxides), scandium, molybdenum, thallium, thorium, cadmium, boron, among other components, have also been suggested for use in oxidative coupling catalysts. See, for instance, United States Patents Nos. 4,450,310; 4,443,646; 4,499,324;4,443,645; 4,443,648; 4,172,810; 4,205,194; 4,239,658; 4,523,050; 4,442,647; 4,499,323; 4,443,644; 4,444,984; 4,659,668; 4,704,487; 4,777,313; 4,780,449; International Patent Publication WO 86/07351, European Patent Applications 189079 (1986); 206042 (1986); 206044 (1986), and 177327 (1985), Australian Patent No. 52925 (1986), Moriyama, et al., "Oxidative Dimerization of Methane Over Promoted MgO, Important Factors," Chem. Soc. Japan, Chem. Lett., 1165 (1986), and Emesh, et al., "Oxidative Coupling of Methane over the Oxides of Groups IIIA, IVA and VA Metals," J. Phys. Chem, Vol. 90, 4785 (1986).

Several researchers have proposed the use of alkali or alkaline earth metals in the form of halides (e.g., chloride, bromide or iodide) in oxidative coupling catalysts. Australian Patent No. 52925 discloses the use of supported calcium chloride, barium bromide, potassium iodide, lithium chloride, cesium chloride, among others for catalysts to oxidatively couple methane to ethane and ethylene. The patentees disclose feeding hydrogen halide to the reaction zone. European Patent Application 210 383 (1986) discloses the addition of gas phase material containing halogen component such as chlorine, methyl chloride and methyl dichloride. Enhanced selectivities are reported when the halogen component is present. The catalysts include those containing one or more of alkali and alkaline earth metal, alkali metal with lanthanide oxide, zinc oxide, titanium oxide or zirconium oxide and others. United States Patent No. 4,654,460 discloses the addition of a halogen-containing material either in the catalyst or via a feed gas in an oxidative coupling process. The catalyst contains one or more alkali metal and alkaline earth metal components. Although no working examples are provided, conversions of methane are said to be increased with halides and selectivities to higher hydrocarbons, particularly ethylene, improved. See also, Burch, et al., "Role of Chlorine in Improving Selectivity in the Oxidative Coupling of Methane to Ethylene," Appl. Catal., Vol. 46, 69 (1989), and "The Importance of Homogeneous Reaction in Oxidative Coupling of Methane Over Chloride Promoted Oxide Catalysts," Catal. Lett., vol. 2, 249 (1989), who propose mechanistic possibilities for the effect of halide in oxidative coupling of methane, and Minachev, et al., "Oxidation Condensation of Methane in a New Pathway to the Synthesis of Ethane, Ethylene, and Other Hydrocarbons," Russ. Chem. Rev., Vol. 57, 221 (1988).

The use of silver in catalysts for oxidative coupling has also been proposed by a number of workers. Australian Patent No. 52925 suggests that oxidative coupling catalysts may contain a wide variety of components, one of which is silver. No working examples are provided demonstrating the use of any silver-containing catalyst nor is there any specific disclosure of silver-containing catalysts or processes for using them. International Patent Application Publication WO 7351 (1986) (United States Patent No. 4,780,449) discloses catalysts for oxidative coupling using a catalyst with oxides of one or more of the following elements: beryllium, magnesium, calcium, strontium, barium, scandium, yttrium, lanthanum and lanthanide series excluding cerium and mixtures thereof. The patent further states that the catalysts can contain promoters of components from Groups IA, IIA, IIIA, lanthanides, IVB, VB, IB and mixtures thereof. Example 22 reports a 1 weight percent silver or lanthanum oxide catalyst which was calcined in air at 600°C for 4 hours. The catalyst was operated at 800°C with a cofeed with 0.30 atm of methane, 0.05 atm oxygen and 0.65 atm argon at a hourly space velocity of 37500 recriprocal hours. The methane conversion is reported to be 20 percent and the selectivity to $C_2$'s is reported to be 59.7 percent. $Li_2O/La_2O_3$ and some other metal oxide/$La_2O_3$ catalysts also exhibited higher methane conversions and selectivities to ethane and ethylene at 800°C than the silver-containing catalyst. Similar catalysts are reported by DeBoy, et al., "Oxidative Coupling of Methane over Alkaline Earth Promoted $La_2O_3$," J. Chem. Soc. Chem. Commun., 982 (1988).

Ungar, et al., High Selectivity Catalysts for the Oxidative Coupling of Methane Complex Oxides with the Rock Salt Structure", Appl. Catal., Vol. 42, L1 (1988). disclose a silver chromate catalyst which provided no conversion to ethylene.

Ueda, et al., "Layered Bismuth Oxyhalides: A New Family of Methane Oxidation Catalysts," Proceedings 9th Int. Cong. on Catalysis, Calgary, Vol. 2, C1 Chemistry, 960 (1988) report catalysts having particular crystal structures. A Ag $Bi_3O_4Cl_2$ catalyst prepared by Ueda, et al., appears to have no advantage in conversion of methane or yield to ethylene and ethane for oxidative coupling over $ABi_3O_4Cl_2$ catalysts wherein A is sodium or potassium. Gaffney, et al, "Oxidative Coupling of Methane over Sodium Promoted Praseodymium Oxide," J. Catal., Vol. 114, 422 (1988) disclose a silver on praseodymium oxide catalyst. The authors describe a 16.4 weight percent silver on praseodymium oxide catalyst. The silver-containing catalyst provided a 41 percent methane conversion and a selectivity to ethane and ethylene of 1 percent. An unpromoted praseodymium oxide catalyst provided a 35 percent conversion with a 2 percent selectivity to the $C_2$'s.

Garnett, et al., "Catalytic Oxidation of Methane Over $AlPO_4$-5 and Metal-Doped $AlPO_4$-5", Bibby, et al., ed., Methane Conversion, Elsevier Science Publishers B.V., Amsterdam (1988), report the use of 3-5 weight percent of platinum, silver, lead, copper, cobalt or vanadium doping on $AlPO_4$-5 molecular sieve for methane oxidation coupling. They conclude that "The metals which enhanced both selectivity and reactivity were Pb and Ag. Pb-$AlPO_4$-5... increased selectivity by approximately 10% overall, while the reactivity of Pb-$AlPO_4$-5 increased slightly (5%). Ag-$AlPO_4$-5... affected the reactivity of the $CH_4$ oxidation resulting in an increased selectivity, while, reactivity was possibly increased by 2%." (p. 392)

Summary of the Invention

By this invention processes are provided for the oxidative coupling of lower alkane to produce heavier hydrocarbons which exhibit enhanced activity for oxidation coupling processes. In the processes of this invention, both the alkane and reactive oxygen-containing material are cofed to a reaction zone, thereby avoiding problems associated with sequential oxidative coupling processes. Moreover, with the lower oxidative coupling temperatures that are achievable using the processes of this invention, enhanced catalyst stability can be obtained.

In accordance with this invention, catalysts are used which comprise silver in elemental or catalytically active combined form in an amount up to about 30, say, up to about 20, weight percent based on the weight of the catalyst which is sufficient to provide enhanced oxidative coupling activity, and a sufficient amount of at least one alkali or alkaline earth metal to enhance the selectivity of the catalyst. The processes are conducted in the presence of halogen-containing vapor phase component.

In the oxidation coupling processes of this invention, lower alkane, e.g., 1 to 3 carbon atoms, and reactive oxygen containing material are fed to a reaction zone containing a catalytically-effective amount of oxidative coupling catalyst in the presence of halogen-containing vapor phase additive; the reaction zone is maintained under oxidative coupling conditions to convert at least a portion of the alkane to higher hydrocarbon; and an effluent comprising higher hydrocarbon produced in the reaction zone is withdrawn.

A halogen-containing vapor phase additive is provided in the feed to the reaction zone in an amount sufficient to enhance at least one of the activity of alkane conversion and the selectivity to heavier hydrocarbon. The halogen-containing vapor phase additive comprises at least one of chlorine, bromium and iodine, either in molecular form or in combined form. The combination of the halogen-containing vapor phase additive and alkali metal and/or alkaline earth metal component enables the activity of the catalyst to be enhanced without undue loss of selectivity due, e.g., to burning reactions caused by the presence of the silver.

Detailed Discussion of the Invention

In accordance with this invention, lower alkane is converted to higher hydrocarbons. The lower alkane preferably comprises at least one of methane, ethane and propane, and because of its abundance and the desire to convert it to higher hydrocarbons, methane is the most preferred component in the feed. The products of the conversion are higher hydrocarbons, especially alkanes and alkenes. Often, the desired conversion products are alkenes of two to four carbon atoms, especially ethylene and propylene. Because of its widespread use in commodity chemicals, product mixtures exhibiting a high selectivity to ethylene are typically preferred. The reaction is conducted in the presence of a reactive oxygen-containing material (oxidizing material) which for the purposes herein means atomic or molecular oxygen or a compound or chemical complex that contains an oxygen atom available for the oxidative coupling.

The hydrocarbon conversion process is conducted in a cofeed, or simultaneous process, in which both the oxidizing material and the alkane-containing feed are provided at the same time to the reaction zone.

In a cofeed process, the oxidizing material and alkane may be introduced by one or more separate streams or, most commonly, in a premixed stream. Generally, the mole ratio of alkane to active oxygen atom of the oxidizing material (an active oxygen atom is an oxygen atom that is available for oxidation) is at least about 1:2, say, about 1:2 to 50:1, preferably 1:1 to 20:1. The alkane typically comprises at least about 2 volume percent, e.g., up to about 95, say, 5 to 90, volume percent of the total gases fed to the reaction zone. Frequently, the feed streams are diluted with essentially inert gases such as helium, nitrogen, argon, steam, and carbon dioxide. When diluted, the diluent usually provides between about 5 to 95 volume percent of the feed streams.

The oxidizing material may be any suitable oxygen-bearing material which, under the conditions in the reaction zone, yields an active oxygen atom for the oxidative coupling. Convenient oxidizing materials are normally gaseous such as molecular oxygen, (e.g., as oxygen, enriched air or air), ozone and gases which yield oxygen such as $N_2O$. Materials that are liquid or solid at ambient conditions may also be used provided that they can be facilely introduced into the reaction zone.

The reaction proceeds at elevated temperatures. Generally, a minimum temperature must be achieved before significant higher hydrocarbon production occurs. If the temperature is too high, an undue amount of the hydrocarbon is consumed in oxidation or degradation reactions. The bulk temperature, i.e., temperature of the reaction zone as opposed to localized temperature which may exist in the reaction zone, is often less than about 800° C. Typically, the temperature is in the range of about 500° to 775° C., and preferably about 600° to 750° C. The reactants are usually preheated prior to their introduction into the reaction zone; for instance, to within about 200° C, preferably about 100° C of the temperature in the reaction zone.

The pressure in the reaction zone may vary widely from less than atmospheric to 100 atmospheres absolute or more, say, 1 to 50, atmospheres absolute.

In general, the reactions proceed rapidly and, hence, the reactants may reside in the reaction zone under reaction conditions for a relatively short period of time, e.g., less than about 20 seconds, often less than about 10 seconds. Frequently, the residence time is about 0.001 to 5, say, 0.1 to 3, seconds. The gas hourly space velocity based on the total gases fed to the reaction zone to the volume of the reaction zone is often about 50 to 50,000, preferably, 500 to 15000, reciprocal hours. Since the alkane conversion reactions do not require the presence of catalyst to proceed, the overall volume of the vessel in which the reaction takes place may be substantially larger than that of the reaction zone containing catalyst. Even so, the volume of the reaction zone is frequently calculated as the volume of the vessel filled with catalyst.

The reaction may be conducted in any suitable reactor capable of providing the reaction temperatures.

The catalysts of this invention are generally characterized as supported, i.e., the silver or silver compound is dispersed on another material which provides the shape to the catalyst. The support, which is catalytically-acceptable, may be catalytically active or inert; however, the preferred supports usually exhibit some catalytic activity.

The support material may comprise refractory oxides, e.g., alumina, zirconia, titania, silica, spinels, perovskites (e.g., $ABO_3$ wherein A is a Group IIA metal and B is a Group IVA metal), aluminosilicates, alkaline earth oxides (e.g., magnesium oxide, calcium oxide, barium oxide and strontium oxide); alkaline earth carbonates (e.g., barium carbonate and strontium carbonate), and the like. Advantageously, the support material has a surface area of at least about 0.1, preferably, at least about 0.2, say, 0.2 to 60 or 100 or more, square meters per gram. (Determined by the nitrogen B.E.T. Method, J. Am. Chem. Soc., Vol. 60, 309 (1938)).

The amount of silver or silver compound is frequently at least about 0.01, e.g., about 0.01 to 30, say about 1 to 2 weight percent (calculated as the metal) based on the total weight of the catalyst. The silver may be initially provided on the catalyst in elemental or combined form (e.g., as a nitrate, oxide, carbonate, amine complex, or chloride). While not wishing to be limited to theory, it is believed that the silver should be maintained in at least a partially chemically combined form (e.g., as in oxide, carbonate, or hydroxide) during the process.

The catalysts contain one or more alkali and alkaline earth metal components. These components are generally present in an atomic ratio to silver of at least 0.0001:1, say, about 0.05:1 to 1000:1, and typically, 0.05:1 to 10:1. These components comprise compounds of one or more of sodium, lithium, potassium, rubidium, cesium, beryllium, magnesium, calcium, barium and strontium.

The catalysts preferably contain at least one Group IA or Group IIA compound. Most preferably, at least one Group IIA component, especially barium and/or strontium, is present. These components may be oxides, hydroxides, or salts, e.g., halides ($X^-$), oxyhalides ($OX^-$), halites ($XO_2^-$), halates ($XO_3^-$), perhalates ($XO_4^-$) (wherein X is one or more of chlorine, bromine and iodine), carbonates, sulfates, molybdates, tungstates, cerates, nitrates, etc. The Group IA and Group IIA components, if not contained in the support, are present in an amount of at least about 0.001, say, about 0.01 to 50, most preferably, at least about 0.1 to 20, weight percent of the total catalyst.

Other adjuvants that may be present in the catalyst include metal oxides, hydroxides and salts of one or more of Group IIIA (including lanthanide series) and Group IVA (e.g., titanium and zirconium) elements. These adjuvants may be used in amount of between 0.0001 and 50 weight percent of the total catalyst.

The supported catalysts may be prepared by any convenient technique. Techniques which have been proposed include coating the catalyst support with a slurry or paste of the ingredients or impregnating the support using a solution or suspension or complex of the ingredients (the impregnation may be simultaneous for all components or sequential). The impregnation may be by an incipient wetness technique or by immersion in the mother liquor or by evaporation of a solvent in a solution or suspension containing the support. The catalysts may be dried and, optionally, calcined.

The catalyst size and configuration may vary depending upon the reactor type. For fluid, ebulating and riser reactors, the catalyst is typically between about 30 and 300 microns in major dimension. In fixed bed reactors, the catalyst may be in any suitable configuration including spheres, pellets, cylinders, monoliths, etc., and the size and shape may be influenced by pressure drop considerations for the gases passing through the bed. Often, the catalyst is at least about 0.2 centimeter, say, about 0.5 to 2 centimeters, in major dimension. Monolithic catalysts, which may comprise a support having the catalytically active component thereon or which may be homogeneous, can be sized to fit the reactor volume.

The vapor phase halogen component is provided to the reaction zone during the process. It may be added intermittently or continuously. The halogen component may be provided as a solid, liquid or vapor when added. The halogen component may be halogen, e.g., chlorine, bromium or iodine, or a halogen-containing compound. The halogen-containing compounds (chlorine, bromine and iodine-containing compound.) may be inorganic or organic such as hydrogen halide, carbon tetrahalide, methylene halide, methyl dihalide, methyl trihalide, ethyl halide, ethyl dihalide, ethyl trihalide, ethyl tetrahalide, vinyl halide, sulfonyl chloride, phosphonyl chloride, etc. Often, the organic halides have from 1 to 3 halogen atoms and 1 to 3 carbon atoms. The amount of halogen component which can be added to the process, can vary; however, the amount added should be sufficient to provide the desired yield of higher hydrocarbon and the sought ethylene to ethane mole ratio. With either too little or too much halogen component addition, the catalyst performance will be adversely effected. Most frequently, if too little or too much halogen component has been added, good performance can be achieved by altering the rate of halogen component addition.

It has been found that when catalysts containing halide salts are first operated, evolution of a halogen-containing component occurs, often providing enhanced performance. However, this effect disapates with time and halogen component is preferably added.

The amount of halogen component to be added for a given catalyst system will depend on the nature of the catalyst. Moreover, the optimum amount may change with the use of the catalyst.

Also, the type of halogen being added will influence the performance of the reaction system. Within these guidelines, the amount of continuous vapor phase addition of the halogen component is often within the range of 0.1 to 5000, say, 1 to 1000, parts per million by volume based on the volume of feed to the reaction zone.

A volatilized metal component may be introduced intermittently or continuously into the reaction zone in an amount sufficient to reduce the rate of vapor phase alkane conversion reaction. Enhancements in selectivities to higher hydrocarbons may be obtained using the volatilized metal component additive, and the selectivity benefits provided by the catalysts better realized. See United States Patent Application Ser. No. (D-16270), filed on even date herewith, hereby incorporated by reference.

In practice, the amount of volatilized metal component to be added can be ascertained by monitoring the selectivity to higher hydrocarbons and adjusting the amount introduced. The amount of volatilized metal component introduced can vary widely and will depend, to some extent, on the nature of the volatilized metal component. Often, the amount of volatilized metal component introduced is at least about 0.001 picogram, say, about 0.005 picogram to 10,000 or more milligrams per cubic meter of alkane in the feed (determined at standard temperature and pressure ("STP")).

It is not essential, and indeed in most instances it is not the case, that the volatilized metal component has a boiling point below the reaction temperature. Most convenient volatilized metal components have boiling points much higher than the reaction temperature under reaction conditions. However, the volatilized metal component should have a vapor pressure under the reaction conditions sufficient to provide the sought amount of volatilized metal component to achieve the sought reduction in vapor phase alkane conversion. Accordingly, the volatilized metal components are preferably molten or near to becoming molten (e.g, withiin $100\,^{\circ}$C) at the reaction temperature. The melting points of some volatilized metal components are set forth in Table I.

TABLE I

| VOLATILIZED COMPONENT | APPROXIMATE MELTING POINT (°C) |
|---|---|
| Barium chloride | 963 |
| Strontium chloride | 875 |
| Barium bromide | 847 |
| Sodium chloride | 801 |
| Calcium chloride | 782 |
| Potassium chloride | 770 |
| Sodium bromide | 758 |
| Barium iodide | 740 |
| Potassium bromide | 730 |
| Rubidium chloride | 718 |
| Potassium iodide | 686 |
| Sodium iodide | 653 |
| Cesium chloride | 645 |
| Strontium bromide | 643 |
| Cesium iodide | 612 |
| Lithium chloride | 605 |
| Barium hydroxide | 408 |
| Potassium hydroxide | 405 |
| Sodium hydroxide | 318 |
| Cesium hydroxide | 272 |

The preferred volatilized metal components are salts of Group IA and Group IIA metals. Salts such as nitrates, chromates, etc., may have explosive characteristics at the reaction temperatures. Thus, these salts and others which may adversely decompose or oxidize are generally avoided. The volatilized metal component, however, may be added in the form of an oxide, hydroxide, peroxide, superoxide or salt and be converted to another compound under the reaction conditions. In general, the preferred salts are halides, especially chlorides, bromides and iodides.

The introduction of the volatilized metal component into the reaction zone may be by any convenient means. Advantageously, the volatilized metal component is relatively uniformly distributed as it passes through the reaction zone. The introduction may be, for instance, by adding a stream of volatilized metal component in the vapor form to the reaction zone or to the feed stream. Since most volatilized metal components are not gases under the reaction conditions, the volatilized metal components must enter the vapor phase through sublimation or the effects of partial pressure over the metal component in the liquid state. Hence, it is often desirable to pass, at an elevated temperature (e.g. 400° to 1000°C, say, 500° to 850°C), all or a portion of the feed gas over the metal component to volatilize a desired amount of the metal component. Since oxidation reactions can occur at these elevated temperatures, frequently the volatilized metal component is contacted with either an alkane-containing stream having an essential absence of oxygen or a diluent gas or an oxygen-containing stream having an essential absence of alkane. The stream can be admixed with the remaining portion of the feed gases (for a continuous process).

In the event that the volatilized metal component solidifies, coalesces or is adsorbed or absorbed in the reaction zone, a build-up of the volatilized metal component may occur. In many instances, the build-up is not unduly deleterious; however, if it adversely affects the performance of the reaction, temporary cessation of the introduction of the volatilized metal component may be indicated.

The following examples are provided by way of illustration of the invention and are not in limitation thereof. All parts and percentages of solids are by weight and of liquids and gases are by volume unless otherwise noted or clear from the context.

EXAMPLE 1

7

Silver catalysts are made by putting silver on a support by one of three general procedures, Procedure X, Procedure Y, or Procedure Z. Additional components are added to small portions of silver on alpha alumina from General Procedures X or Y as described below.

In Procedure X, a 9 weight percent silver on alpha aluminum oxide catalyst is made as follows. 600 Grams of 88% aqueous lactic acid are added to a glass beaker and heated to 80°C with stirring (30 minutes). Silver oxide (220 grams available from Ames Goldsmith Corporation, Glens Falls, New York) is slowly added, with stirring. The mixture is stirred an additional 30 minutes at 80-85°C, then 10 milliliters of CP grade hydrogen peroxide are added. The clear solution is slowly added over a period of 5 minutes via a 1000 milliliter glass separatory funnel to 300 grams of alpha-aluminum oxide (Norton Company, Akron, Ohio SA5502 hollow cylinder supports). The support is first evacuated under a vacuum of from less than kPa to 70 kPa (absolute) pressure at 80°C for 120 minutes in a 5.7 centimeters inside diameter (5.9 cm o.d.) glass tube, which is 36 centimeters. This tube is fitted with a stopcock on the bottom and on the top, through a two-hole, black rubber, size 12 stopper, with a separatory funnel and a vacuum adapter. The glass tube is heated with a 6 foot Thermodyne heat tape, controlled by a Variac (TM) power controller. The impregnating solution remains in the tube with the support at about 81°C for 60 minutes, then is drained. Recovered catalysts are placed in an oven at 60°C and atmospheric pressure for 2 hours, then roasted on a moving belt at 500°C using a forced air flow. The procedure for belt roasting catalysts which is used in Procedures X and Y is described in United States Patent No. 4,419,276, hereby incorporated by reference. The catalysts are then ground to 30 to 60 mesh (U. S. Sieve Series) prior to evaluation or addition of other components.

In procedure Y, a 20 weight percent silver on an alpha aluminum oxide catalyst is made using the procedure described in United States Patent No. 4,419,276. The silver oxide is available from either Ames Goldsmith Corporation, Glen Falls, New York, or Metz Metallurgical Co. Norton 5451 alpha aluminum oxide, 50 grams, 30 to 60 mesh (U. S. Sieve Series) is used as support.

In Procedure Z, a 16.9 weight percent silver on barium carbonate catalyst is made using Procedure Y except that barium carbonate is used instead of alpha aluminum oxides.

For those catalysts with additional components, these are added by one of two general procedures, Procedure A or Procedure B. In Procedure A, supported catalysts are prepared using the incipient wetness technique. In this procedure, the amounts of components required to give the desired loading are dissolved in a quantity of deionized, distilled water necessary to just fill the pores of the support. The solution is then added to the support particles. In some cases, if the dopants are not easily soluble, suspensions of the components are added to the support. The resulting material is dried in a vacuum oven at 130°C under a vacuum of 16-84 kPA for 1 to 50 hours (usually 16 - 20 hours). The dried catalysts are either tested without further treatment or first calcined in air. Metal or inorganic compound loadings are expressed as weight percent of elemental metal based on 100 weight percent catalyst.

In Procedure B, supported catalysts are prepared by adding the proper amount of component(s) to a mixture of water which is stirred with the support, while heating in a glass container on a hotplate for 2 to 3 hours (or until almost no water is left), to distribute the material in and on the support. If the components are not easily soluble, they are finely ground to assist in solubilizing or suspending the component. Deionized, distilled water is used (50 mL unless stated otherwise). The resulting material is dried in a vacuum oven at 130°C under a vacuum of 16-84 kPa for 1 to 50 hours (preferred 16-20 hours). The dried catalysts are then calcined in air. Metal or inorganic compound loadings are expressed as weight percent based on 100 weight percent support.

Catalyst I: A catalyst of 9 weight percent silver (calculated as elemental silver) on aluminum oxide is prepared by General Procedure X.
Approximately 330.16 grams of catalyst are obtained.

Catalyst II: A catalyst of 1.0 weight percent potassium chloride (calculated as elemental potassium based on 100 weight percent catalyst) and 9 weight percent silver on aluminum oxide, Catalyst I, is prepared by General Procedure X followed by General Procedure A using 0.10 grams of potassium chloride (from Johnson Matthey/AESAR Group, Seabrook, NH, Lot number S96353, 99.999 weight percent) in 0.98 grams of deionized water and 5 grams of Catalyst I. The catalyst particle size is 30 to 60 mesh (U. S. Sieve Series).

Catalyst III: A catalyst of 0.18 weight percent lithium chloride (calculated as elemental lithium) and 9 weight percent silver on aluminum oxide, Catalyst I, is prepared by General Procedure X followed by General Procedure A using 0.0579 grams of lithium chloride (from Johnson Matthey/AESAR Group, Seabrook, NH, Lot number S94408, 99.999 weight percent) in 0.98 grams of deionized water and 5 grams of Catalyst I. The catalyst particle size is 30 to 60 mesh (U. S. Sieve Series).

Catalyst IV: A catalyst of 3.5 weight percent barium chloride (calculated as elemental barium) and 9 weight percent silver on aluminum oxide, Catalyst I, is prepared by General Procedure X followed by General Procedure A using 0.279 grams of barium chloride (from Johnson Matthey/AESAR Group, Seabrook, NH, Lot number S94730, 99.99999 weight percent) in 0.98 grams of deionized water and 5 grams of Catalyst I. The catalyst particle size is 30 to 60 mesh (U. S. Sieve Series).

Catalyst V: A catalyst of 0.61 weight percent sodium chloride (calculated as elemental sodium) and 9 weight percent silver on aluminum oxide, Catalyst I, is prepared by General Procedure X followed by General Procedure A using 0.078 grams of sodium chloride (from Johnson Matthey/AESAR Group, Seabrook, NH, Lot number S95696, 99.999 weight percent) in 0.98 grams of deionized water and 5 grams of Catalyst I. The catalyst particle size is 30 to 60 mesh (U. S. Sieve Series).

Catalyst VI: A catalyst of 3.4 weight percent cesium chloride (calculated as elemental cesium) and 8.7 weight percent silver on aluminum oxide, Catalyst I, is prepared by General Procedure X followed by General Procedure A using 0.2257 grams of cesium chloride (from Johnson Matthey/AESAR Group, Seabrook, NH, Lot number S94942, 99.999 weight percent) in 0.98 grams of deionized water and 5 grams of Catalyst I. The catalyst particle size is 3 to 60 mesh (U.S. Sieve Series).

Catalyst VII: A catalyst of 2 weight percent silver (calculated as elemental silver on aluminum oxide is prepared by General Procedure Y. Approximately 62.5 grams of catalyst are obtained. The catalyst particle size is 30 to 60 mesh (U.S. Sieve Series).

Catalyst VIII: A catalyst of 16.9 weight percent silver (calculated as elemental silver) on barium carbonate (from Johnson Matthey/AESAR Group, Seabrook, NH, Lot number S97090R, 99.9997 weight percent) is prepared by General Procedure Z. The catalyst is calcined at 800°C for 4 hours. Approximately 18.82 grams of catalyst are obtained. The catalyst particle size is 30 to 60 mesh (U.S. Sieve Series).

Catalyst IX: A catalyst of 0.10 weight percent hydrated praseodymium nitrate (calculated as elemental praseodymium) and 20 weight percent silver on aluminum oxide is prepared by General Procedure Y followed by General Procedure A using 0.018 gram of hydrated praseodymium nitrate (6 waters of hydration, from Johnson Matthey/AESAR Group, Seabrook, NH, Lot number 11240A, 99.99 weight percent) in 1.17 grams of deionized water and 6 grans of Catalyst VII. The catalyst is calcined at 800°C for 4 hours. The catalyst particle size is 30 to 60 mesh (U. S. Sieve Series).

Catalyst X: A catalyst of 0.01 weight percent hydrated praseodymium nitrate (calculated as elemental praseodymium) and 20 weight percent silver on aluminum oxide is prepared by General Procedure Y followed by General Procedure A using 0.00185 grams of hydrated praseodymium nitrate (6 waters of hydration, from Johnson Matthey/AESAR Group, Seabrook, NH, Lot number 11240A, 99.99 weight percent) in 1.17 grams of deionized water and 6 grams of catalyst VII. The catalyst is calcined at 800°C for 4 hours. The catalyst particle size is 30 to 60 mesh (U. S. Sieve Series).

Catalyst XI: A catalyst of 0.10 weight percent bismuth nitrate (calculated as elemental bismuth) and 20 weight percent silver on aluminum oxide is prepared by General Procedure Y followed by General Procedure A using 0.0139 grams of bismuth nitrate (from Johnson Matthey/AESAR Group, Seabrook, NH, Lkot number S96494, 99.999 weight percent) in 2.17 grams of deionized water and 6 grams of Catalyst VII except that the solution is at a slightly elevated temperature such that it remains clear. The catalyst is calcined at 800°C for 4 hours. The catalyst particle size is 30 to 60 mesh (U. S. Sieve Series).

Catalyst XII: A catalyst of 0.10 weight percent barium nitrate (calculated as elemental barium) and 20 weight percent silver on aluminum oxide is prepared by General Procedure Y followed by General Procedure A using 0.011 grams of barium nitrate (J. T. Baker Chemical Co., Phillipsburg, NJ, Lot number 43774, 99.3 weight percent) in 1.17 grams of deionized water and 6 grams of Catalyst VII. The catalyst is calcined at 800°C for 4 hours. The catalyst particle size is 30 to 60 mesh (U. S. Sieve Series).

Catalyst XIII: A catalyst of 1.0 weight percent barium nitrate (calculated as elemental barium) and 20 weight percent silver on aluminum oxide is prepared by General Procedure Y followed by General Procedure A using 0.11 grams of barium nitrate (J. T. Baker Chemical Co., Phillipsburg, NJ, Lot number 43774, 99.3 weight percent) in 1.17 grams of deionized water and 6 grams of Catalyst VII except that the solution is at a slightly elevated temperature such that it remains clear. The catalyst is calcined at 800°C for 4 hours. The catalyst particle size is 30 to 60 mesh (U. S. Sieve Series).

Catalyst XIV: A catalyst of 1.0 weight percent potassium nitrate (calculated as elemental potassium) and 20 weight percent silver on aluminum oxide is prepared by General Procedure Y followed by General Procedure A using 0.1551 grams of potassium nitrate (from Johnson Matthey/AESAR Group, Seabrook, NH, LKot number S95682, 99.999 weight percent) in 1.17 grams of deionized water and 6 grams of Catalyst VII. The catalyst is calcined at 800°C for 4 hours. The catalyst particle size is 30 to 60 mesh (U. S. Sieve Series).

Catalyst XV: A catalyst of 19 weight percent silver (calculated as elemental silver) on aluminum oxide is prepared by General Procedure Y. Approximately 61.89 grams of catalyst are obtained.

Catalyst XVI: A catalyst of 3.5 weight percent barium chloride (calculated as elemental barium) and 18 weight percent silver on aluminum oxide is prepared by General Procedure Y followed by General Procedure A using 0.335 grams of barium chloride (from Johnson Matthey/AESAR Group, Seabrook, NH, Lot number S94730, 99.9999 weight percent) in 1.17 grams of deionized water and 6 grams of Catalyst XV. The catalyst is calcined at 800°C for 4 hours. The catalyst particle size is 30 to 60 mesh (U. S. Sieve Series).

Catalyst XVII. A catalyst of 3.4 weight percent barium chloride (calculated as elemental barium), 3.4 weight percent lanthanum oxide (calculated as elemental lanthanum) and 18 weight percent silver on aluminum oxide is prepared by General Procedure Y followed by General Procedure B using 0.335 grams of barium chloride (from Johnson Matthey/LAESAR Group, Seabrook, NH, Lot number S94730, 99.9999 weight percent) and 0.262 grams of lanthanum oxide (from Johnson Matthey/AESAR Group, Seabrook, NH, Lot number R3677, 99.99 weight percent) in 30 milliliters of deionized water and 6 grams of Catalyst XV. the catalyst is calcined at 800°C for 4 hours. The catalyst particle size is 30 to 60 mesh (U. S. Sieve Series).

Catalyst XVII: A catalyst of 3.5 weight percent barium chloride (calculated as elemental barium), 0.35 weight percent lanthanum oxide (calculated as elemental lanthanum) and 18 weight percent silver on aluminum oxide is prepared by General Procedure Y followed by General Procedure B using 0.335 grams of barium chloride (from Johnson Matthey/AESAR Group, Seabrook, NH, Lot number S94730, 99.9999 weight percent) and 0.0262 grams of lanthanum oxide (from Johnson Matthey/AESAR Group, Seabrook, NH, Lot number R3677, 99.99 weight percent) in 30 milliliters of deionized water and 6 grams of Catalyst XV. The catalyst is calcined at 800°C for 4 hours. The catalyst particle size is 30 to 60 mesh (U. S. Sieve Series).

Catalyst XIX: A catalyst of 4.1 weight percent barium chloride (calculated as elemental barium), 4.1 weight percent lanthanum oxide (calculated as elemental lanthanum) and 9 weight percent silver on aluminum oxide, Catalyst I, is prepared by General Procedure X followed by General procedure B using 0.335 grams of barium chloride (from Johnson Matthey/AESAR Group, Seabrook, NH, LKot number S94730, 99.99999 weight percent) and 0.262 grams of lanthanum oxide (from Johnson Matthey/AESAR Group, Seabrook, NH, Lot number R3677, 99.999 weight percent) in 30 milliliters of deionized water and 6 grams of Catalyst I. The catalyst is calcined at 800°C for 4 hours. The catalyst particle size is 30 to 60 mesh (U. S. Sieve Series).

Catalyst XX: A catalyst of 4.3 weight percent barium chloride (calculated as elemental barium) and 9 weight percent silver on aluminum oxide, is prepared by General Procedure X followed by General Procedure A using 0.335 grams of barium chloride (from Johnson Matthey/AESAR Group, Seabrook, NH, Lot number S94730, 99.9999 weight percent) on 1.21 grams of deionized water and 6 grams of Catalyst I. The catalyst is calcined at 800°C for 4 hours. The catalyst particle size is 30 to 60 mesh (U. S. Sieve Series).

Catalyst XXI: A catalyst of 1.1 weight percent hydrated calcium chloride (calculated as elemental calcium) and 19 weight percent silver on aluminum oxide is prepared by General Procedure Y followed by General Procedure A using 0.353 grams of hydrated calcium chloride (Johnson Matthey/AESAR Group, Seabrook, NH. Lot number LV01629KV, 99.99+ weight percent, hydration not specified) in 1.17 grams of deionized water and 6 grams of Catalyst XV. The catalyst particle size is 30 to 60 mesh Reactor System (U. S. Sieve Series).

The catalysts are evaluated using a reactor system containing two parallel reactors to which may be fed a variety of gases. The reactor system consists of a gas feed system, two tubular microreactors (a steam feed system to one of these), two Lindberg tube furnaces, two liquid collection systems, an on-line gas analyzer with a stream selector, and a separate liquids analyzer.

Two Lindberg Mini-Mite (TM), model 55035, tube furnaces (800 watts, 115 volts, 1100°C maximum operating temperature) are used for heating the tubular microreactors. The entire cylindrical heated chamber is comprised of two halves, making up the split-hinge furnace design. Each half is a composite unit of Moldatherm (TM), Lindberg's high temperature ceramic fiber insulation, and a helically coiled alloy heating element.

Quartz reactors are used. These include Reactor C and Reactor D. Reactors are operated vertically.

Reactor C is constructed of 0.9 centimeter inside diameter quartz tubing (1.1 centimeters o. d.). Reactor C is 56.5 centimeters long. The bottom of the heated region of the reactor is 12.75 centimeters above the bottom of the reactor. The top of the heated region is 46.75 centimeters above the bottom of the reactor. The post-catalyst volume is packed with 20/40 or 14/30 mesh (U. S. Sieve Series) quartz chips in order to minimize the high temperature post-catalyst residence time of the products, and to hold the catalyst in place. The bottom end of the reactor is connected to 1.0 centimeter inside diameter (1.2 centimeters o. d.) quartz tubing at a right angle to the reactor. The ends of the reactor are made of quartz "O"-ring joints (1.5 centimeters i. d. at the inlet and 0.9 cm i. d. at the outlet) which allow easy placement of the reactor into the system.

The center of the catalyst bed is 18.5 cm above the bottom of the oven, or 31.25 centimeters above the bottom of the reactor. When the center of the reactor is heated to 802° C under typical flow rates of gases, the bottom of the heated portion of the reactor is at 645° C.

The top 30.5 centimeters of Reactor D is constructed of 0.9 centimeters inside diameter quartz tubing (1.1 cm o. d.). This is connected to 26 centimeters of 3 millimeters inside diameter (5 mm o. d. ) quartz tubing. Reactor D is 56.5 centimeters long. The bottom of the heated region of the reactor is 12.75 centimeters above the bottom of the reactor. The top of the heated region is 43.75 centimeters above the bottom of the reactor. The post-catalyst volume of the wider part of the reactor is packed with 20/40 or 14/30 mesh quartz chips, quartz wool, or both quartz chips and quartz wool, in order to minimize the high temperature post-catalyst residence time of the products, and to hold the catalyst in place. The bottom end of the reactor is connected to 1.0 centimeter inside diameter (1.2 cm o. d.) quartz tubing at a right angle to the reactor.

The center of the catalyst bed is 18.5 centimeters above the bottom of the oven, or 31.25 centimeters above the bottom of the reactor. Reactor D exhibits a similar temperature profile to that of Reactor C.

The catalyst bed is formed in Reactors by providing quartz chips within the range of 14 to 40 mesh (U. S. Sieve Series) below and above the catalyst bed, which is separated from the quartz chips by quartz wool. The length of the catalyst bed along the reactor varies, depending on the weight of catalyst used, the density of the catalyst, and the type of reactor used. Most catalysts are within the range of 30 to 100 mesh (U. S. Sieve Series), with many from 30 to 60 mesh and many from 60 to 100 mesh.

In the general operating procedure, the reactors are filled with washed quartz chips above and below the catalyst bed. Those portions of Reactor D with tubing of 0.3 centimeters i. d. are not filled with quartz. Quartz wool is used to separate quartz chips from the catalyst bed and to hold quartz chips in the reactor. Undiluted catalysts of varying mesh sizes are used except in cases where the catalyst is only available as a fine powder. In these cases, it is diluted with quartz chips within the 14 to 40 mesh range.

Charged reactors are pressurized under nitrogen to about 176 kPa then flushed with nitrogen during heating. Gas mixtures entering and exiting the reactor system during reactions are analyzed at intervals using gas chromatography. The reactor pressure is maintained at about 176 kPa throughout the reaction, and nitrogen is the diluent gas. After experiments, the reactant flow is terminated and the reactor is flushed with nitrogen while cooling.

EXAMPLES 2-21

In Examples 2-21, about 5 grams of catalyst are used with 10 volume percent methane, 5 volume percent oxygen, and 85 volume percent nitrogen. Catalysts I-VI are tested in Reactor C and Catalysts VII-XXI are tested in Reactor D. The results are summarized in Table I. In the Table, the following defined terms are used. Dashes are used when amounts are too small to be accurately measured.

| Temperature (Temp) | Temperature of the catalyst bed (° C). |
|---|---|
| Time | Time since reactant flow is started (minutes). |
| $CH_4$ ($CH_4C$) | Mole % of methane reacted. |
| Sel | Selectivity to ethane + ethylene expressed as a percent, based on methane reacted. |
| GHSV | Gas hourly space velocity (reciprocal hours) at (STP) |
| ECl | Ethyl chloride feed rate in parts per million by volume based on total feed. |

## TABLE I

| Example | Catalyst | GHSV hr$^{-1}$ | CH$_4$ C % | C$_2$Sel % | Temp °C | ECl ppmv | Time Min. |
|---|---|---|---|---|---|---|---|
| 2 | I | 2182 | 6.5 | ———— | 400 | 50 | 185 |
| | | 1636 | 25.0 | ———— | 425 | 50 | 305 |
| | | 1091 | 24.4 | ———— | 600 | 50 | 5830 |
| | | 1091 | 31.5 | 19.6 | 700 | 50 | 5880 |
| | | 1091 | 30.0 | 13.2 | 700 | 50 | 5915 |
| | | 341 | 30.5 | 17.5 | 700 | 50 | 6245 |
| | | 341 | 25.5 | 2.4 | 700 | 50 | 7100 |
| | | 2182 | 25.9 | 1.7 | 700 | 50 | 7280 |
| 3 | II | 750 | 2.0 | 0.4 | 500 | 50 | 130 |
| | | 750 | 16.8 | 23.0 | 650 | 50 | 410 |
| | | 750 | 22.3 | 32.2 | 675 | 50 | 1450 |
| | | 750 | 32.4 | 33.1 | 700 | 50 | 1795 |
| | | 900 | 30.1 | 33.9 | 700 | 50 | 1855 |
| | | 900 | 30.8 | 33.6 | 700 | 50 | 2055 |
| | III | 682 | 2.2 | 76.9 | 400 | 50 | 35 |
| | | 682 | 18.7 | 8.0 | 600 | 50 | 185 |
| | | 682 | 29.1 | 11.7 | 650 | 50 | 260 |
| 5 | IV | 682 | 3.6 | 45.6 | 500 | 50 | 150 |
| | | 682 | 11.9 | 21.8 | 600 | 50 | 365 |
| | | 682 | 32.1 | 32.1 | 650 | 50 | 440 |
| | | 682 | 29.5 | 30.6 | 650 | 50 | 590 |
| | | 682 | 33.6 | 30.2 | 675 | 50 | 1420 |
| | | 409 | 34.2 | 30.3 | 675 | 50 | 1565 |
| | | 1773 | 33.6 | 27.2 | 700 | 50 | 1755 |
| 6 | V | 682 | 18.1 | 23.9 | 650 | 50 | 405 |
| | | 682 | 13.8 | 30.2 | 650 | 50 | 1265 |
| | | 682 | 25.5 | 34.0 | 675 | 50 | 1385 |
| | | 682 | 25.9 | 34.2 | 675 | 50 | 1455 |
| | | 1364 | 14.6 | 36.0 | 675 | 50 | 1530 |
| | | 409 | 34.4 | 32.3 | 675 | 50 | 1620 |
| | | 1091 | 28.8 | 37.4 | 700 | 50 | 1710 |
| | | 818 | 35.0 | 33.5 | 700 | 50 | 1790 |
| | | 2182 | 18.9 | 26.9 | 700 | 50 | 7160 |

12

TABLE I (Continued)

| | | | | | | | |
|---|---|---|---|---|---|---|---|
| 7 | VI | 682 | 15.9 | 1.8 | 600 | 50 | 220 |
| | | 682 | 23.7 | 10.1 | 650 | 50 | 305 |
| | | 682 | 18.2 | 10.3 | 650 | 50 | 440 |
| | | 682 | 30.2 | 24.2 | 700 | 50 | 1240 |
| | | 1364 | 23.3 | 23.6 | 700 | 50 | 1315 |
| 8 | VII | 2341 | 20.4 | 0.0 | 425 | 0 | 365 |
| | | 2341 | 1.3 | 0.0 | 425 | 5 | 540 |
| | | 2341 | 10.1 | 0.0 | 475 | 0 | 7705 |
| 9 | VIII | 2341 | 25.3 | 0.0 | 400 | 0 | 4080 |
| | | 2341 | 24.9 | 0.0 | 400 | 0 | 4115 |
| | | 2341 | 17.8 | 0.0 | 350 | 0 | 4250 |
| | | 2341 | 26.6 | 0.0 | 350 | 10 | 4340 |
| | | 2341 | 2.7 | 0.0 | 350 | 10 | 4430 |
| | | 2341 | 26.8 | 0.3 | 600 | 10 | 5410 |
| | | 2341 | 26.1 | 0.2 | 550 | 500 | 6725 |
| | | 2341 | 26.1 | 0.2 | 650 | 0 | 30 |
| 10 | IX | 2341 | 14.7 | 0.0 | 400 | 0 | 300 |
| | | 2341 | 26.7 | 0.0 | 425 | 0 | 350 |
| | | 2341 | 15.9 | 0.0 | 400 | 10 | 3830 |
| | | 2341 | 4.1 | 0.0 | 400 | 10 | 4080 |
| | | 2341 | 26.6' | 0.2 | 650 | 10 | 4190 |
| 11 | X | 2341 | 26.1 | 0.0 | 600 | 0 | 270 |
| | | 2341 | 22.4 | 0.0 | 575 | 0 | 325 |
| | | 2341 | 24.5 | 0.0 | 550 | 10 | 3865 |
| 12 | XI | 2341 | 7.3 | 0.0 | 650 | 0 | 145 |
| | | 2341 | 22.6 | 1.3 | 700 | 0 | 200 |
| | | 2341 | 23.4 | 1.3 | 700 | 10 | 1255 |
| | | 2341 | 24.5 | 1.0 | 700 | 50 | 1660 |
| 13 | XII | 2341 | 26.1 | 0.0 | 450 | 0 | 170 |
| | | 2341 | 16.0 | 0.0 | 400 | 0 | 265 |
| | | 2341 | 5.3 | 0.0 | 400 | 10 | 1310 |
| | | 2341 | 26.5 | 0.7 | 700 | 10 | 1630 |
| | | 2341 | 27.8 | 0.7 | 700 | 50 | 1705 |

## TABLE I (Continued)

| | | | | | | | |
|---|---|---|---|---|---|---|---|
| 14 | XIII | 2341 | 22.0 | 0.0 | 400 | 0 | 215 |
| | | 2341 | 19.8 | 0.0 | 400 | 50 | 270 |
| | | 2341 | 27.4 | 0.6 | 700 | 50 | 1385 |
| | | 2341 | 26.0 | 1.7 | 700 | 500 | 1540 |
| 15 | XIV | 2341 | 23.1 | 0.4 | 600 | 0 | 180. |
| | | 2341 | 19.7 | 0.3 | 625 | 0 | 245 |
| | | 2341 | 14.8 | 0.3 | 625 | 50 | 310 |
| | | 2341 | 0.0 | 11.1 | 625 | 50 | 1325 |
| | | 2341 | 26.3 | 0.6 | 700 | 50 | 1460 |
| | | 2341 | 17.2 | 23.3 | 700 | 500 | 1565 |
| 16 | XVI | 1263 | 17.2 | 25.4 | 670 | 5 | 255 |
| | | 1263 | 16.7 | 24.9 | 670 | 5 | 305 · |
| | | 1263 | 27.6 | 2.3 | 670 | 50 | 410 |
| | | 1263 | 27.1 | 0.4 | 670 | 5 | 1490 |
| 17 | XVII | 2526 | 6.3 | 20.2 | 650 | 5 | 220 |
| | | 1263 | 26.7 | 15.3 | 670 | 5 | 325 |
| | | 1263 | 27.9 | 0.9 | 670 | 5 | 415 |
| 18 | VVIII | 1263 | 15.8 | 28.0 | 670 | 5 | 290 |
| | | 1263 | 26.2 | 0.4 | 670 | 50 | 1265 |
| | | 1263 | 27.1 | 0.3 | 670 | 5 | 1445 |
| 19 | XIX | 1171 | 16.9 | 27.4 | 650 | 5 | 135 |
| | | 1171 | 26.6 | 27.2 | 675 | 5 | 280 |
| | | 1171 | 30.8 | 11.9 | 675 | 50 | 1330 |
| | | 1171 | 27.1 | 30.4 | 675 | 500 | 4105 |
| 20 | XX | 1171 | 14.1 | 28.3 | 650 | 5 | 165 |
| | | 1171 | 22.8 | 28.7 | 675 | 5 | 235 |
| | | 1171 | 27.2 | 0.8 | 675 | 5 | 1180 |
| | | 1171 | 26.2 | 5.5 | 675 | 500 | 1495 |
| | | 1171 | 21.3 | 30.4 | 675 | 500 | 4060 |
| 21 | XXI | 1263 | 6.1 | 14.4 | 600 | 5 | 55 |
| | | 1263 | 15.8 | 25.4 | 650 | 5 | 115 |
| | | 1263 | 15.2 | 24.8 | 650 | 5 | 185 |
| | | 1263 | 29.0 | 3.4 | 700 | 5 | 280 |

## Claims

1. A process for oxidative coupling of alkane of 1 to 3 carbon atoms to heavier hydrocarbon comprising feeding the alkane, reactive oxygen-containing material and halogen-containing vapor phase additive in an amount sufficient to enhance at least one of conversion of alkane and selectivity to higher hydrocarbon to a reaction zone containing a catalytically-effective amount of oxidative coupling catalyst, said oxidative coupling catalyst being a supported catalyst comprising a catalytically-active amount of at least one alkali metal or alkaline earth metal compound sufficient to enhance selectivity to heavier hydrocarbon and silver and/or silver compound in an amount sufficient to enhance oxidative coupling activity; maintaining the reaction zone under oxidative coupling conditions to convert at least a portion of the alkane to heavier hydrocarbon, and withdrawing from the reaction zone an effluent containing heavier hydrocarbon produced

14

in the reaction zone.

2. The process of claim 1 wherein the reactive oxygen-containing material comprises oxygen.

3. The process of claim 2 wherein the catalyst comprises Group IIA metal compound.

4. The process of claim 3 wherein the catalyst comprises at least one of barium compound and strontium compound.

5. The process of claim 4 wherein at least one barium and/or strontium compound comprises at least one of oxide, peroxide, hydroxide, carbonate, chloride, bromide and iodide.

6. The process of claim 4 wherein barium and/or strontium compound comprises 0.1 to 20 weight percent of the catalyst.

7. The process of claim 2 wherein the halogen-containing vapor phase additive comprises at least one of hydrogen halide, organic halide of 1 to 3 carbon atoms and halogen, wherein the halide or halogen is at least one of chlorine, bromine and iodine.

8. The process of claim 7 wherein the halogen-containing vapor phase additive is provided in an amount of about 1 to 1000 ppm based on the feed to the reaction zone.

9. The process of claim 7 wherein the oxidative coupling conditions comprise a temperature between about 500°C and 775°C and a gas hourly space velocity of between about 500 and 15000 reciprocal hours.

10. The process of claim 9 wherein the mole ratio of alkane to oxygen atom is about 1:1 to 20:1.

11. The process of claim 10 wherein the halogen-containing vapor phase additive comprises chlorine-containing compound.

12. The process of claim 3 wherein the catalyst comprises silver compound.

13. The process of claim 3 wherein the support comprises at least one of catalytically active Group IA or Group IIA metal compounds.

14. The process of claim 3 wherein the support comprises alumina.

15. The process of claim 3 wherein the support comprises titanium dioxide.

16. The process of claim 2 wherein the silver and/or silver compound is present in an amount of between 0.1 and 20 weight percent based (calculated as the metal) on the total weight of the catalyst.

15

# DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| P,X | WO-A-9 000 532 (QUANTUM CHEMICAL CORP.) <br> * Claims * | 1-2 | C 07 C 2/84 |
| X | US-A-4 465 893 (OLAH) <br> * Claims * | 1,7 | |
| X | US-A-4 654 459 (SOFRANKO) <br> * Claims * | 4-5 | |
| X | DE-A-3 534 530 (M. BAERNS) <br> * Claims * | 4-5 | |
| A | DE-A-3 503 664 (AKZO) <br> * Claims * | 1,4 | |
| A | CHEMICAL ABSTRACTS, vol. 108, no. 21, May 1988, page 648, abstract no. 186163g, Columbus, Ohio, US; <br> & JP-A-62 267 243 (IDEMITSU KOSAN CO., LTD) 19-11-1987 | 4-5 | |

| TECHNICAL FIELDS SEARCHED (Int. Cl.5) |
|---|
| C 07 C 2/00 |

The present search report has been drawn up for all claims

| Place of search | Date of completion of search | Examiner |
|---|---|---|
| The Hague | 28 November 90 | VAN GEYT J.J.A. |